(19) Europäisches Patentamt
European Patent Office
Office européen des brevets

(11) Veröffentlichungsnummer: **0 025 932**
**A1**

(12) # EUROPÄISCHE PATENTANMELDUNG

(21) Anmeldenummer: **80105375.2**

(22) Anmeldetag: **09.09.80**

(51) Int. Cl.³: **C 07 C 102/00**
C 07 C 103/58
//C07C103/737

(30) Priorität: **19.09.79 DE 2937815**

(43) Veröffentlichungstag der Anmeldung:
**01.04.81 Patentblatt 81/13**

(84) Benannte Vertragsstaaten:
**AT BE CH DE FR GB IT LI LU NL SE**

(71) Anmelder: **BASF Aktiengesellschaft**
**Carl-Bosch-Strasse 38**
**D-6700 Ludwigshafen(DE)**

(72) Erfinder: **Halbritter, Klaus, Dr.**
**Schwarzwaldstrasse 19**
**D-6800 Mannheim 1(DE)**

(54) **Verfahren zur Herstellung von 3,3-Dimethyl-pent-4-en-säureamiden.**

(57) Die vorliegende Erfindung betrifft ein Verfahren zur Herstellung von 3,3-Dimethyl-pent-4- en-säureamiden der Formel I

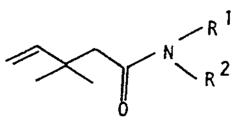 I

ind der
$R^1$ und $R^2$ gleich oder verschieden sein können und einen Alkylrest mit 1 bis 4 Kohlenstoffatomen bedeuten oder zusammen mit dem Stickstoffatom, dessen Substituengen sie sind, einen 5- oder 6-gliedrigen gesättigten Ring bilden, der noch ein weiteres Heteroatom enthalten kann, durch Umsetzung von Acetamidacetalen bzw. Ketenacetalaminalen, mit 3-Methyl-but-2-en-1-ol. Die Umsetzung wird bei einer Temperatur im Bereich zwischen 80 und 220°C gegebenenfalls in Gegenwart eines inerten organischen Lösungsmittels durchgeführt. Die Verfahrensprodukte sind wertvolle Zwischenprodukte für die Herstellung insektizid wirksamer Cyclopropancarbonsäureester.

EP 0 025 932 A1

Croydon Printing Company Ltd

Verfahren zur Herstellung von 3,3-Dimethyl-pent-4-en-säureamiden

Die vorliegende Erfindung betrifft ein Verfahren zur Herstellung von 3,3-Dimethyl-pent-4-en-säureamiden durch Umsetzung von Acetamidacetalen oder Ketenacetalen mit 3-Methyl-but-2-en-1-ol.

Es ist bekannt, daß 3,3-Dimethyl-pent-4-en-säureamide durch Umsetzung von 3,3-Dimethyl-acrylsäureamiden mit Acetaldehyd in Gegenwart eines Peroxids zu 3,3-Dimethyl-3-acetyl-acetamiden und anschließende Reduktion und Dehydratisierung erhalten werden können (JP-OS 77/83 411). Außerdem können 3,3-Dimethyl-pent-4-en-säureamide in an sich üblicher Weise aus 4-Cyano-3,3-dimethyl-but-1-en oder 3,3-Dimethyl-pent-4-en-säure hergestellt werden (DE-OS 27 32 213).

Es wurde gefunden, daß man 3,3-Dimethyl-pent-4-en-säureamide der Formel I

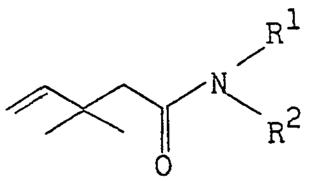

I

in der

$R^1$ und $R^2$ gleich oder verschieden sein können und einen Alkylrest mit 1 bis 4 Kohlenstoffatomen bedeuten oder zusammen mit dem Stickstoffatom, dessen Substituenten sie sind, einen 5- oder 6-gliedrigen gesättigten Ring bilden, der noch ein weiteres Heteroatom enthalten kann, in vorteilhafter Weise erhält, wenn man Acetamidacetale der Formel II

H/BL

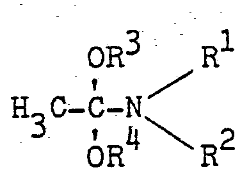

II

in der $R^3$ und $R^4$ gleich oder verschieden sein können und einen Alkylrest mit 1 bis 4 Kohlenstoffatomen bedeuten oder zusammen einen gegebenenfalls verzweigten Alkylenrest mit 2 oder 3 Kohlenstoffatomen bilden und $R^1$ und $R^2$ die oben genannten Bedeutungen haben,

oder Ketenacetalaminale der Formel III

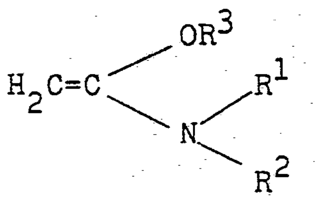

III

in der $R^3$ einen Alkylrest mit 1 bis 4 Kohlenstoffatomen bedeutet und $R^1$ und $R^2$ die oben genannten Bedeutungen haben, mit 3-Methyl-but-2-en-1-ol bei einer Temperatur im Bereich zwischen 80 und 200°C umsetzt.

Die Umsetzung von Acetamidacetalen bzw. Ketenacetalaminalen mit Allylalkoholen ist im Prinzip bekannt. Für die Umsetzung 3,3-dialkylsubstituierter Allylalkohole, wie Geraniol, ist jedoch mehrstündiges Erhitzen der Komponenten auf 150°C erforderlich (Helv. Chim. Acta 47, 2425 bis 2429) (1964), Helv. Chim. Acta 52, 1031 (1969)).

Da jedoch andererseits damit zu rechnen ist, daß das bei höheren Temperaturen instabile 3-Methyl-but-2-en-1-ol bei

140°C dehydratisiert und in Diene, Oligomere und Polymere übergeht bzw. in das beim erfindungsgemäßen Verfahren nicht einsetzbare 3-Methyl-but-3-en-1-ol umgelagert wird (Canad. J. Chem. 47, 4455-4458 (1969)), war es keineswegs zu erwarten, daß die Umsetzung des 3-Methyl-but-2-en-1-ols mit Acetamidacetalen bzw. Ketenacetalaminalen bei höheren Temperaturen ohne Bildung von Nebenprodukten und mit ausgezeichneten Ausbeuten verläuft.

Nach dem erfindungsgemäßen Verfahren lassen sich 3,3-Dimethyl-pent-4-en-säureamide der Formel I herstellen, bei denen $R^1$ und $R^2$ gleich oder verschieden sind und unverzweigte oder verzweigte Alkylreste mit 1 bis 4 Kohlenstoffatomen, wie Methyl, Äthyl, n-Propyl, i-Propyl oder einen Butylrest, bedeuten. $R^1$ und $R^2$ können dabei auch zusammen mit dem Stickstoffatom, dessen Substituenten sie sind, einen 5- oder 6-gliedrigen gesättigten Ring bilden, der noch ein weiteres Heteroatom enthalten kann, beispielsweise einen Pyrrolidin-, Piperidin- oder Morpholinring.

Die Säureamide der Formel I sind wertvolle Zwischenprodukte für die Synthese von insektiziden Wirkstoffen. Sie lassen sich mit Tetrahalogenmethanen in Gegenwart eines Katalysators zu 6,6,6,4-Tetrahalogen-3,3-dimethylhexansäureamiden umsetzen, die wiederum durch Behandlung mit Basen in 2-(2,2-Dihalogenvinyl)-3,3-dimethyl-cyclopropancarbonsäureamide überführt werden können (DE-OS 27 32 213).

Als Ausgangsstoffe kommen Acetamidacetale der Formel II in Betracht, bei denen $R^1$ und $R^2$ die oben genannten Bedeutungen haben und die Substituenten $R^3$ und $R^4$ gleich oder verschieden sind und unverzweigte oder verzweigte Alkylreste mit 1 bis 4 Kohlenstoffatomen, wie Methyl, Äthyl, n-Propyl, i-Propyl oder einen Butylrest, bedeuten. $R^3$ und

$R^4$ können auch zusammen einen gegebenenfalls verzweigten Alkylenrest mit 2 oder 3 Kohlenstoffatomen, wie Ethylen, Trimethylen oder Propylen, bilden. Als Ausgangsstoffe geeignete Ketenacetalaminale sind Verbindungen der Formel III, bei denen die Substituenten $R^1$ und $R^2$ die oben genannten Bedeutungen haben, und $R^3$ einen unverzweigten oder verzweigten Alkylrest mit 1 bis 4 Kohlenstoffatomen, wie Methyl, Äthyl, n-Propyl, i-Propyl oder einen Butylrest, bedeutet. Auch Gemische aus einem Acetamidacetal und einem Ketenacetalaminal können verwendet werden, vorausgesetzt, daß $R^1$ und $R^2$ in Formel II die gleichen Bedeutungen wie in Formel III haben.

Die Ausgangsstoffe der Formel II bzw. III sind zum Teil bekannt und lassen sich in Anlehnung an bekannte Verfahren herstellen (Ann. Chem. 641, 1-39 (1961); Chem. Ber. 96, 1350 bis 1355 (1963)).

Das Mengenverhältnis der Ausgangsstoffe kann in weiten Bereichen variiert werden; zweckmäßigerweise setzt man 0,9 bis 1,3 Mol 3-Methyl-but-2-en-1-ol pro Mol Acetamidacetal der Formel II oder Ketenacetalaminal der Formel III bzw. deren Gemisch ein.

Die Umsetzung wird diskontinuierlich oder kontinuierlich, drucklos oder unter Druck bei Temperaturen im Bereich zwischen 80 und 200°C durchgeführt. Vorzugsweise arbeitet man in einem Temperaturbereich von 130 bis 160°C.

Die Umsetzung verläuft glatt und mit hohen Ausbeuten sowohl in Abwesenheit als auch in Gegenwart eines Lösungsmittels. Geeignete inerte organische Lösungsmittel sind aliphatische und aromatische Kohlenwasserstoffe, wie Xylole, Mesitylen, Chlorbenzol, Nitrobenzol, Tetralin, Decalin; acyclische oder cyclische Äther, wie Di-n-butyläther,

Diäthylenglykoldiäthyläther, Dioxan, Tetrahydrofuran, Diphenyläther, Anisol, sowie Tetramethylensulfon, Dimethylformamid, Dimethylacetamid, Tetramethylharnstoff und Benzonitril.

Zur Durchführung des erfindungsgemäßen Verfahrens wird das Reaktionsgemisch aus 3-Methyl-but-2-en-1-ol und Acetamidacetal bzw. Ketenacetalaminal und gegebenenfalls Lösungsmittel im allgemeinen innerhalb von 20 Minuten bis 3 Stunden von Raumtemperatur auf die gewünschte Umsetzungstemperatur aufgeheizt. Der dabei vor der eigentlichen Reaktion durch Umacetalisierung bzw. Ketenacetalaminalbildung entstehende Alkohol der Formel $R^3OH$ bzw. $R^4OH$ wird zweckmäßigerweise durch Destillation entfernt. Das Endprodukt kann in hoher Reinheit durch Destillation über eine kurze Kolonne gewonnen werden. Für weitere Umsetzungen kann auch der Reaktionsaustrag direkt eingesetzt werden, so daß die destillative Abtrennung entfällt.

Das erfindungsgemäße Verfahren wird durch die folgenden Beispiele erläutert.

Beispiel 1

Zu 180 g eines Gemisches aus 72,5 Gew.% N,N-Dimethylacetamid-dimethylacetal und 27,5 Gew.% 1-Dimethylamino-1-methoxyäthylen gibt man 147 g 3-Methyl-but-2-en-1-ol und bringt die Temperatur innerhalb von 80 Minuten auf 144°C, wobei das gebildete Methanol gleichzeitig aus dem Reaktionsgemisch abdestilliert wird. Anschließend wird 100 Minuten lang auf 144 bis 154°C erhitzt. Die Destillation über eine kurze Kolonne liefert 205 g (90 %) 3,3-Dimethyl-pent-4-en-säure-N,N-dimethylamid vom Sdp. 70°C/2,6 mbar, entsprechend einer Ausbeute von 90 % d.Th..

$C_9H_{17}NO$ (155)

| | C | H | N | O |
|---|---|---|---|---|
| Ber.: | 69,6 | 11,0 | 9,0 | 10,3 |
| Gef.: | 69,3 | 10,8 | 9,1 | 10,6 |

$^1$H-NMR (in $d_6$-DMSO):
1,15 ppm (S), 6 Protonen,
2,30 ppm (S), 2 Protonen,
2,88 ppm (S), 3 Protonen,
2,96 ppm (S), 3 Protonen;
4,82 ppm (D; $J_{cis}$ = 10 Hz), 1 Proton;
4,85 ppm (D; $J_{trans}$ = 18 Hz), 1 Proton;
5,85 ppm (DD); $J_{cis}$ = 10 Hz, $J_{trans}$ = 18 Hz), 1 Proton.

Beispiel 2

Zu 359,7 g eines Gemisches, das 87,6 Gew.% N,N-Dimethylacetamid-dimethylacetal und 7,3 Gew.% 1-Dimethylamino--1-methoxyäthylen enthält, gibt man 279,1 g 3-Methyl--but-2-en-1-ol und bringt die Temperatur innerhalb von 60 Minuten auf 144°C. Anschließend wird diese Temperatur noch weitere 120 Minuten gehalten. Während des Aufheizens und bei der Endtemperatur wird das sich bildende Methanol direkt über eine kurze Kolonne abdestilliert. Bei der anschließenden Destillation über eine 1/2 m-Kolonne erhält man zunächst bei 37 bis 55°C/0,8 mbar 58,8 g eines Vorlaufs, der ca. 7,9 Gew.% 3,3-Dimethyl-pent-4-ensäure-N,N-dimethylamid enthält, und anschließend bei 55 bis 62°C/0,8 mbar 392,3 g 98,2 gewichtsprozentiges 3,3-Dimethyl-pent-4-ensäure-N,N-dimethylamid. In dem verbleibenden Rückstand von 9,8 g sind 80,7 Gew.% Produkt enthalten, so daß sich die Gesamtausbeute an 3,3-Dimethyl-pent-4--ensäure-N,N-dimethylamid auf 97,7 % d.Th. beläuft. (Der Gehalt an 3,3-Dimethyl-pent-4-ensäure-N,N-dimethylamid kann gaschromatographisch bestimmt werden.)

Patentansprüche

1. Verfahren zur Herstellung von 3,3-Dimethyl-pent-4-en--säureamiden der Formel I

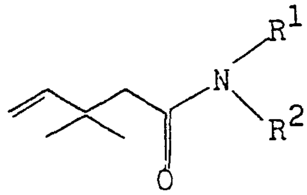

I

in der
$R^1$ und $R^2$ gleich oder verschieden sein können und einen Alkylrest mit 1 bis 4 Kohlenstoffatomen bedeuten oder zusammen mit dem Stickstoffatom, dessen Substituenten sie sind, einen 5- oder 6-gliedrigen gesättigten Ring bilden, der noch ein weiteres Heteroatom enthalten kann, dadurch gekennzeichnet, daß man Acetamidacetale der Formel II

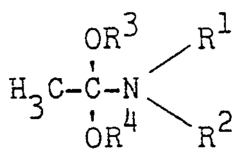

II

in der
$R^3$ und $R^4$ gleich oder verschieden sein können und einen Alkylrest mit 1 bis 4 Kohlenstoffatomen bedeuten oder zusammen einen gegebenenfalls verzweigten Alkylenrest mit 2 oder 3 Kohlenstoffatomen bilden und $R^1$ und $R^2$ die oben genannten Bedeutungen haben,

oder Ketenacetalaminale der Formel III

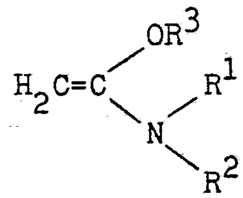 III,

in der

$R^3$ einen Alkylrest mit 1 bis 4 Kohlenstoffatomen bedeutet und $R^1$ und $R^2$ die oben genannten Bedeutungen haben,

mit 3-Methyl-but-2-en-1-ol bei einer Temperatur im Bereich zwischen 80 und 200°C umsetzt.

2. Verfahren nach Anspruch 1, dadurch gekennzeichnet, daß man 3-Methyl-but-2-en-1-ol mit einem Gemisch aus einem Acetamidacetal der Formel II und einem Ketenacetalaminal der Formel III, wobei $R^1$ und $R^2$ in Formel II die gleichen Bedeutungen wie in Formel III haben, umsetzt.

3. Verfahren nach Anspruch 1, dadurch gekennzeichnet, daß man die Umsetzung in Gegenwart eines inerten organischen Lösungsmittels durchführt.

4. Verfahren nach Anspruch 1, dadurch gekennzeichnet, daß man die Umsetzung bei einer Temperatur im Bereich zwischen 130 und 160°C durchführt.

0025932

Europäisches Patentamt

# EUROPÄISCHER RECHERCHENBERICHT

Nummer der Anmeldung

EP 80 10 5375

| EINSCHLÄGIGE DOKUMENTE | | | KLASSIFIKATION DER ANMELDUNG (Int Cl.3) |
|---|---|---|---|
| Kategorie | Kennzeichnung des Dokuments mit Angabe, soweit erforderlich, der maßgeblichen Teile | betrifft Anspruch | |
| | JOURNAL OF ORGANIC CHEMISTRY, Band 37, Nr. 23, 17. November 1972, R.K. HILL et al.: "Asymmetric induction in the thermal reactions of allylic alcohols with N,N-dimethylacetamide dimethyl acetal and triethyl orthoacetate", Seiten 3737-3740<br>* Seiten 3738-3739 *<br>-- | 1-4 | C 07 C 102/00<br>103/58//<br>C 07 C 103/737 |
| | CHEMISCHE BERICHTE, 104. Jahrgang, Nr. 11, 1971, W. SUCROW et al.: "Stereochemie der Claisen-Umlagerung mit 1-dimethylamino-1-methoxy-propen-(1)" Seiten 3679-3688<br>* Seiten 3680,3682 *<br>-- | 1-4 | RECHERCHIERTE SACHGEBIETE (Int. Cl.3)<br>C 07 C 102/00<br>103/58 |
| | DE - A - 2 602 508 (HOFFMANN-LA ROCHE)<br>* Ansprüche; Beispiele 3,4 *<br>---- | 1-4 | |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 05-12-1980 | MOREAU |